# EUROPEAN PATENT SPECIFICATION

(11) **EP 2 292 174 B1**
(45) Date of publication and mention of the grant of the patent: **12.06.2013**
(21) Application number: 10180579.4
(22) Date of filing: 27.07.2004
(51) Int. Cl.: A61B 18/22, A61F 9/008, A61B 1/07, G02B 6/38, A61B 19/00

(54) **Illumination source**
Beleuchtungsquelle
Source d'illumination

(30) Priority: 28.07.2003 US 490399 P; 05.03.2004 US 550979 P; 05.06.2004 US 577740 P; 05.06.2004 US 577618 P; 27.07.2004 US 900939
(43) Date of publication of application: 09.03.2011
(62) Divisional of application: 04786120.8
(73) Proprietor: Synergetics, Inc., O'Fallon, MO 63368 (US)
(72) Inventor: Auld, Michael D., Flemingtom NJ 08822 (US); Easley, James C., O'Fallon, MO 63368 (US); Kane, Jonathan S., Hudson, NH 03051 (US); Scheller, Gregg, Wildwood, MO 63038 (US)
(74) Representative: Hano, Christian

(56) References cited:
- WO-A1-95/34011
- US-B1- 6 367 958

## Description

The art of the present invention relates to fiberoptic endoscopic probes for vitreoretinal surgery in general and more particularly to an illumination source comprising a single illumination source having an output of illumination light transmitted through one or more lenses and focused upon an optical fiber.

Prior art vitreoretinal surgical procedure utilizes discrete and separate optical fibers for the delivery of typically non-coherent light for illumination and coherent laser beam light for surgical treatment of tissues. Although prior art "illuminated laser probes" of various configurations have been developed, they all utilize separate optical fiber or fibers for the non-coherent illumination stream and the coherent laser delivery. The aforesaid fibers are typically arranged side by side inside of a common needle lumen. An embodiment of this prior art technology is found in US 5 323 766 A. This prior art technology requires a larger or more than one incision in order to introduce illumination and laser treatment light into the eye or other structure, thereby generating greater trauma to the surgical site.

Prior art devices typically utilize a laser deliver core optical fiber diameter of typically 200 to 300 microns since said diameter provides the surgical laser burn spot size most commonly desired by the surgeon. The aforesaid prior art devices have been unable to provide sufficient surgically useful illumination (non-coherent white light) power through such a small fiber, primarily due to the prior art's inability to focus said non-coherent surgically useful light onto such a small spot size. Moreover, none of the prior art devices have combined the aforesaid surgically useful illumination and laser treatment light and transmitted through a single fiber, especially of the aforesaid small size.

WO 95/34011 A1 discloses an apparatus for dimming white light received by an optical fiber, the apparatus being a cover having a thin edge and a flat surface for application to the optical fiber commencing at the fiber rim.

US 6 367 958 B1 discloses an endoscopic device including a dimming assembly which has an array of segments which is successively insertable into a path of a light beam. The segments have outlines of different geometric shapes defining a configuration of a transmission region depending upon an angular position of the shaft.

The object of the invention is to provide an illumination source being capable of dimming an output light from a fiber without affecting the color or aperture of the output.

This object is achieved by an illumination source comprising a single illumination source having an output of illumination light transmitted through one or more lenses and focused upon an optical fiber, and a dimming mechanism comprising a mount on one or more of said lenses which is capable of steering one or more of said lenses whereby a peak illumination of said illumination light is not centered on said optical fiber during dimming.
The dimming mechanism of the illumination source according to the present invention moves the focal point of a dimming lens in order to provide dimming without introducing artifacts, chromatic aberrations, or changes of color temperature.

Where provided herein, dimensions, geometrical attributes, and thread sizes are for preferred embodiment informational and enablement purposes. Alternative embodiments may utilize a plurality of variations of the aforesaid without departing from the scope of the present invention. The art of the present invention may be manufactured from a plurality of materials, including but not limited to metals, plastics, glass, ceramics, or composites.

### BRIEF DESCRIPTION OF THE DRAWINGS

Numerous other objects, features, and advantages of the invention should now become apparent upon a reading of the following detailed description taken in conjunction with the accompanying drawings, in which:

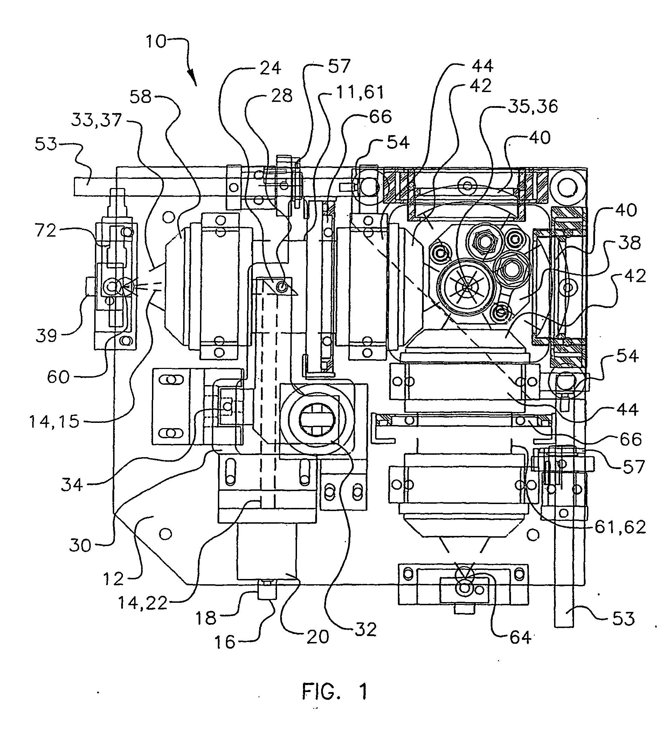

Fig. 1 is a top plan view of a preferred embodiment of the coaxial illuminated laser endoscopic probe and active numerical aperture control apparatus showing illumination and laser light paths without the phototoxicity card, power meter, and ferrule connectors .

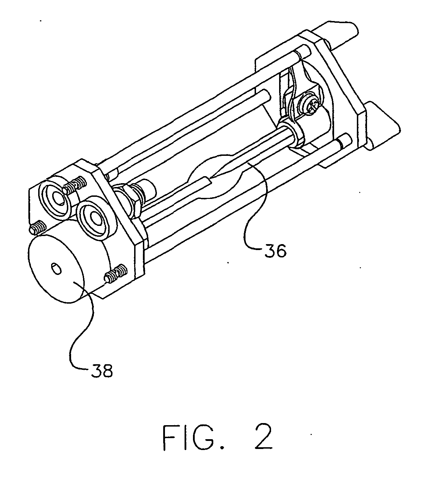

Fig. 2 is a perspective view of the arc lamp source and mount.

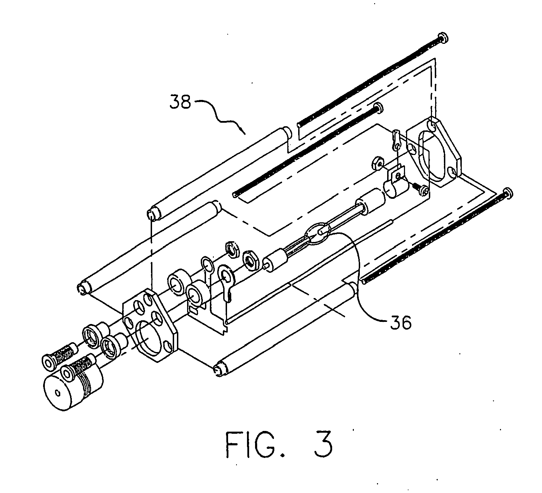

Fig. 3 is an assembly view of the arc lamp source and mount.

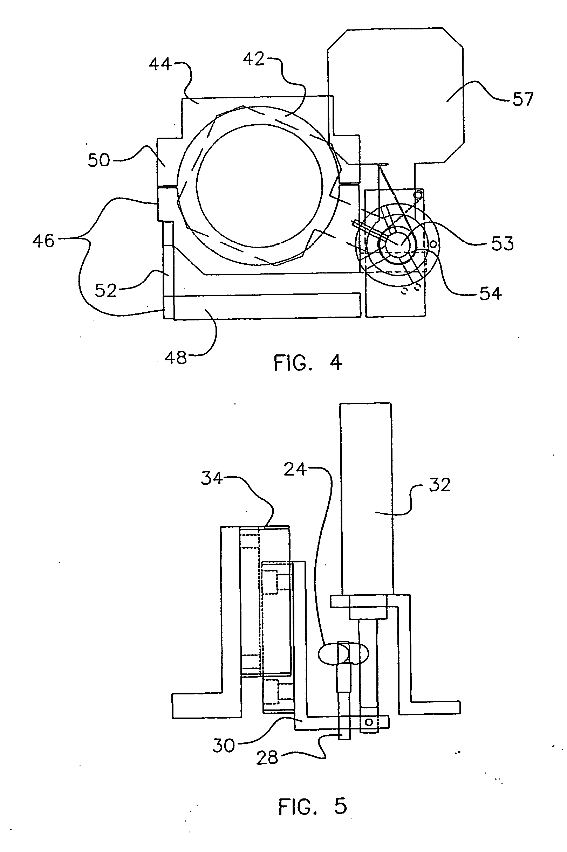

Fig. 4 is a front side plan view of the first lens mount, shaft mounted cam, and shutter with a closed position shutter shown in phantom.
Fig. 5 is a front side plan view of the steering mirror, post, bracket, ball slide, and solenoid in a non-energized extended position.

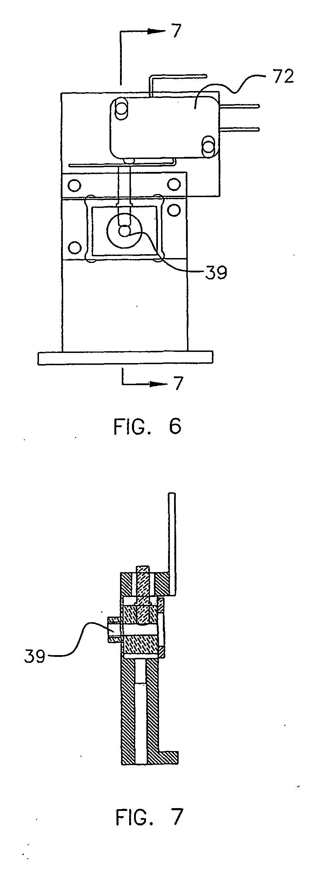

Fig. 6 front side plan view of the first output for laser and illumination light and the switch for sensing the recess in the alignment barrel.
Fig. 7 is a cross sectional view taken along line 7 - 7 of Fig. 6 without the switch body attached.

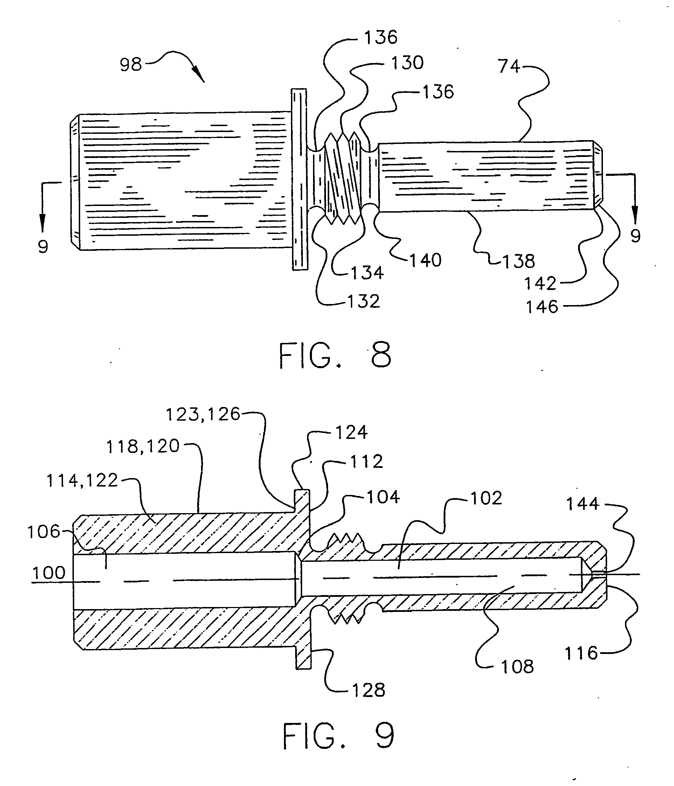

Fig. 8 is a side plan view of the ferrule connector without the recess for preferrably laser and illumination use.
Fig. 9 is a cross sectional view taken along line 9 - 9 of Fig. 8.

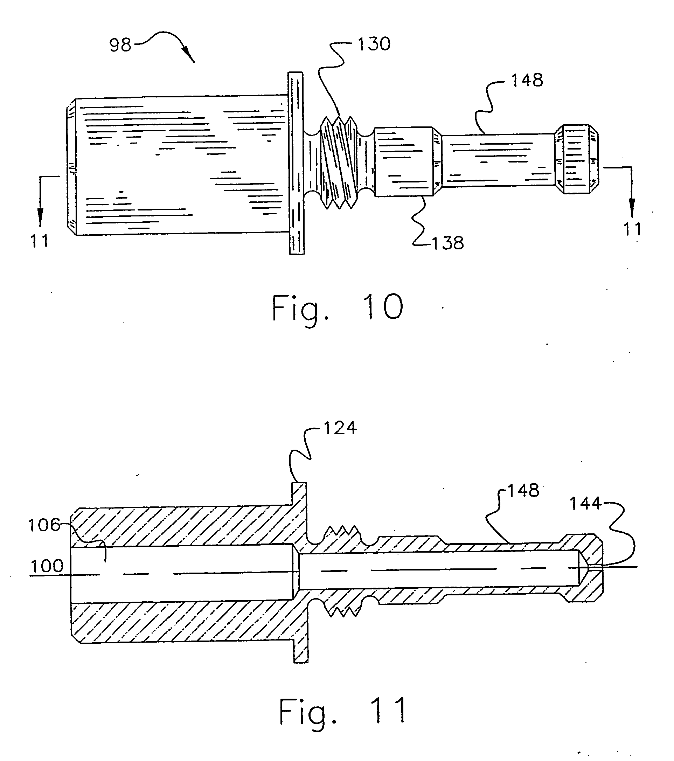

Fig. 10 is a side plan view of the ferrule connector with the recess for preferably illumination use.
Fig. 11 is a cross sectional view taken along line 11 - 11 of Fig. 10.

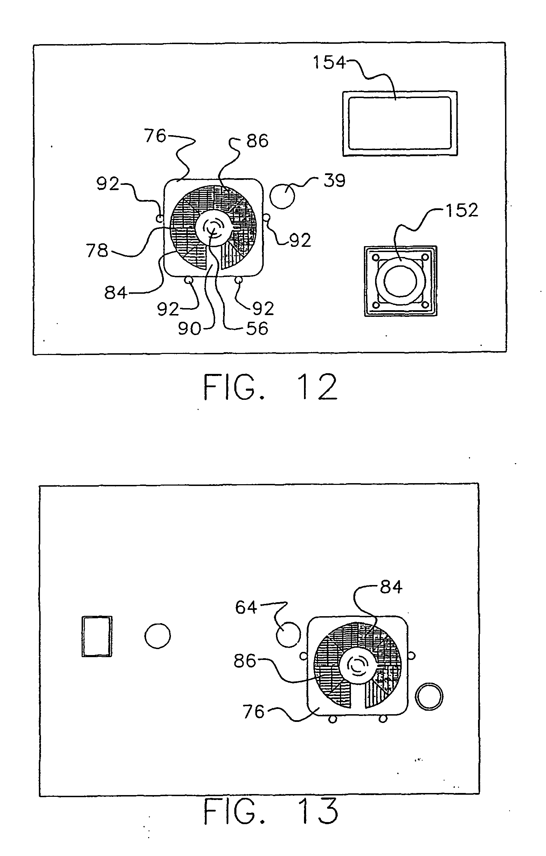

Fig. 12 is a front side plan view of the front panel of the coaxial illuminated laser endoscopic probe and active numerical aperture control apparatus housing showing the first output, illumination level control knob, photoxicity risk card, and laser power meter display and sensor.
Fig. 13 is a right side plan view of the right panel of the coaxial illuminated laser endoscopic probe and active numerical aperture control apparatus housing showing the second output, illumination level control knob, laser connector, power and laser switches, and photoxicity risk card.

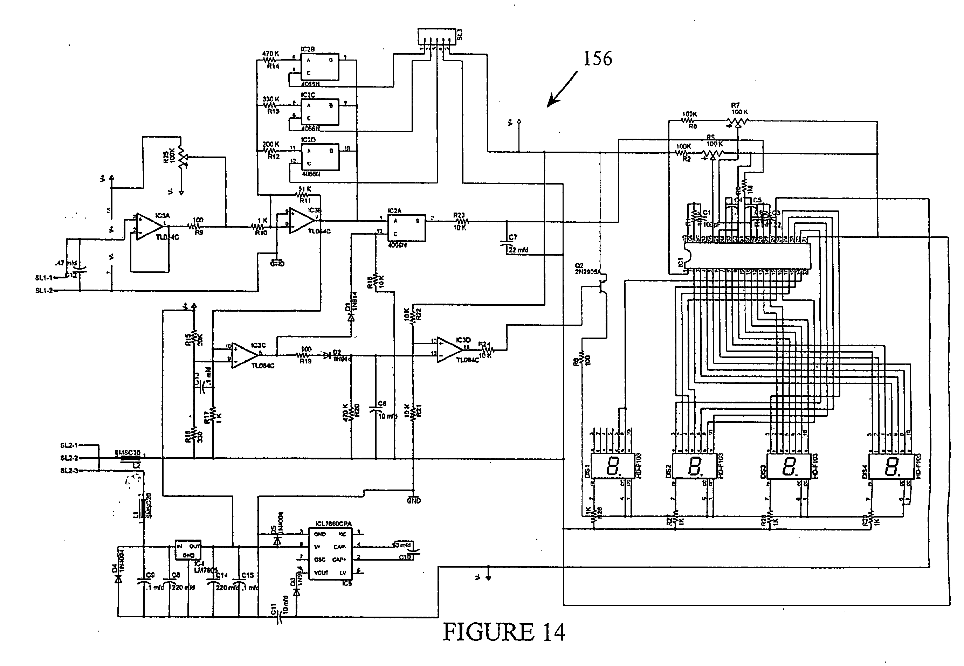

Fig. 14 is an electronic schematic diagram of the laser power meter circuitry.

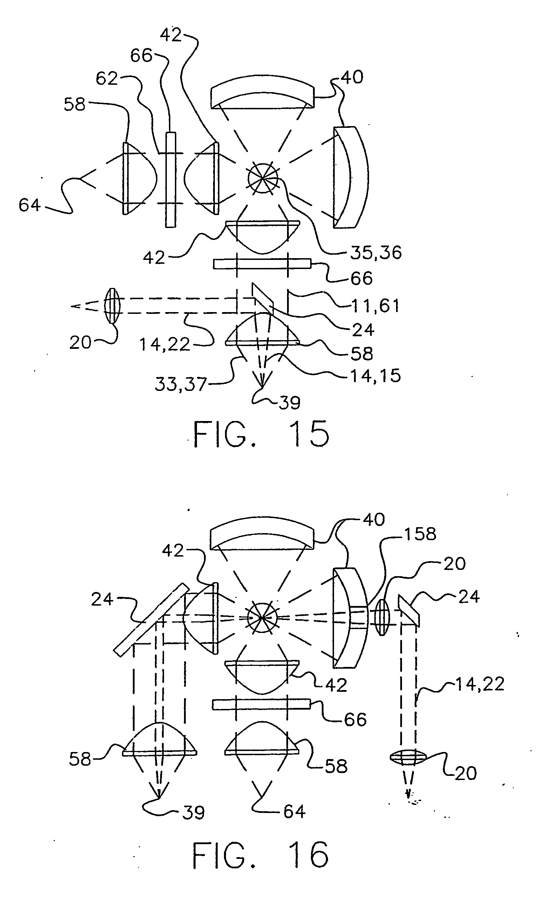

Fig. 15 is an optical schematic diagram of the preferred embodiment of the coaxial illuminated laser endoscopic probe and active numerical aperture control apparatus showing laser and illumination rays, reflectors, mirrors, and lenses.
Fig. 16 is an optical schematic diagram of an alternate embodiment of the coaxial illuminated laser endoscopic probe and active numerical aperture control apparatus showing laser and illumination rays, reflectors, mirrors, and lenses.

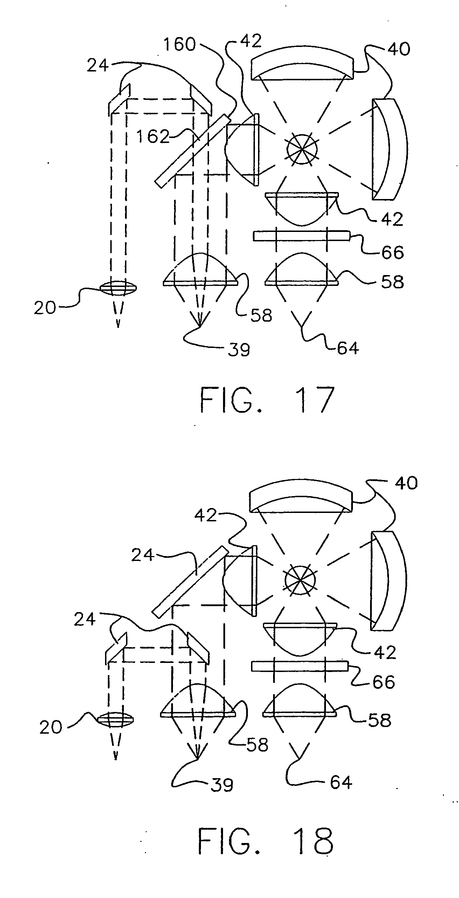

Fig. 17 is an optical schematic diagram of a further alternate embodiment of the coaxial illuminated laser endoscopic probe and active numerical aperture control apparatus showing laser and illumination rays, reflectors, mirrors, and lenses.
Fig. 18 is an optical schematic diagram of another alternate embodiment of the coaxial illuminated laser endoscopic probe and active numerical aperture control apparatus showing laser and illumination rays, reflectors, mirrors, and lenses.

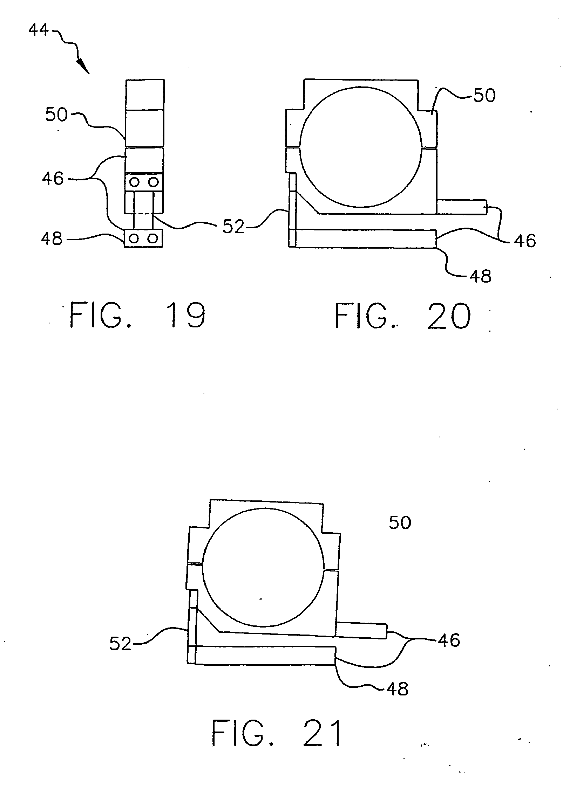

Fig. 19 shows a left side plan view of the first lens mount.
Fig. 20 shows a front side plan view of the first lens mount at a full intensity position
Fig. 21 shows a front side plan view of the first lens mount at a dimmed intensity position.

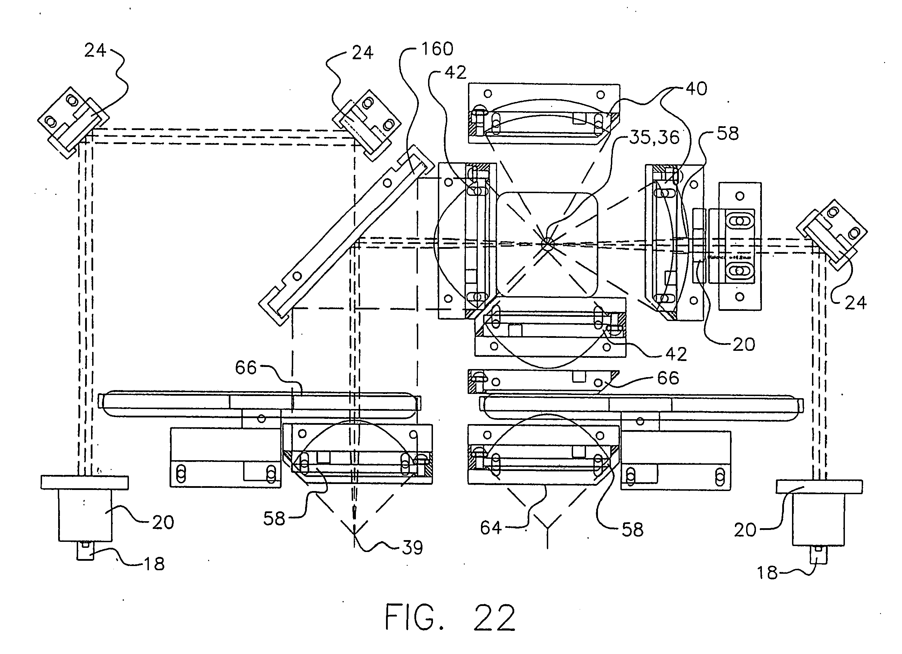

Fig. 22 shows a top plan view of an implementation of the alternate embodiments of the coaxial illuminated laser endoscopic probe and active numerical aperture control apparatus as shown in the optical schematics of Figs. 16 & 17 showing illumination and laser light paths without the phototoxicity card, power meter, and ferrule connectors.

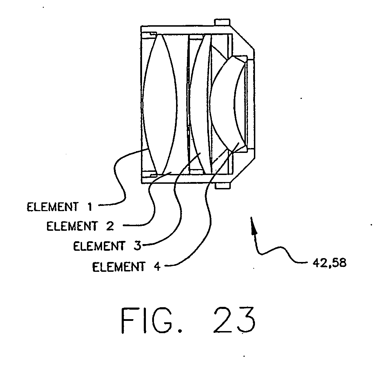

Fig. 23 shows a side plan half cross sectional view of the preferred embodiment of the first and second lenses which correct for color, spherical aberration, and coma and have a back focus 20mm from the apex of the last element and a numerical aperture of .5.

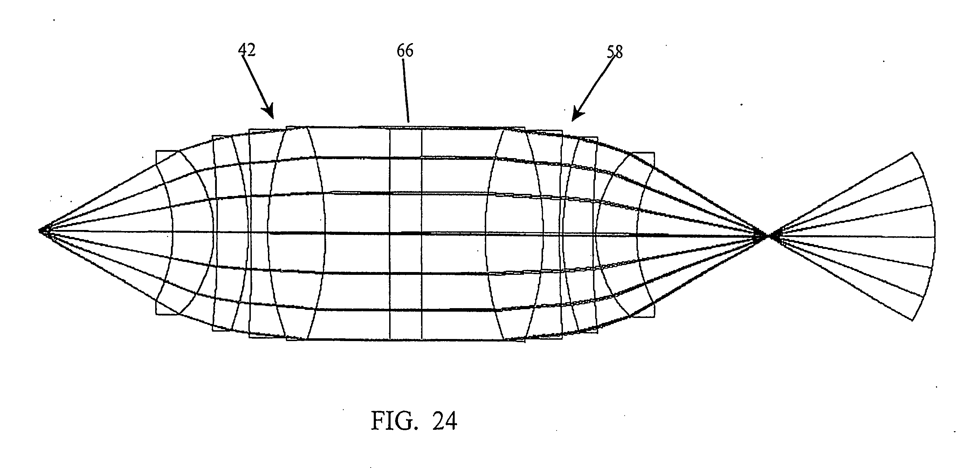

Fig. 24 shows an optical schematic of the first lens set, collimated space, dichroic hot mirror filter, and second lens set with illumination light path rays shown.

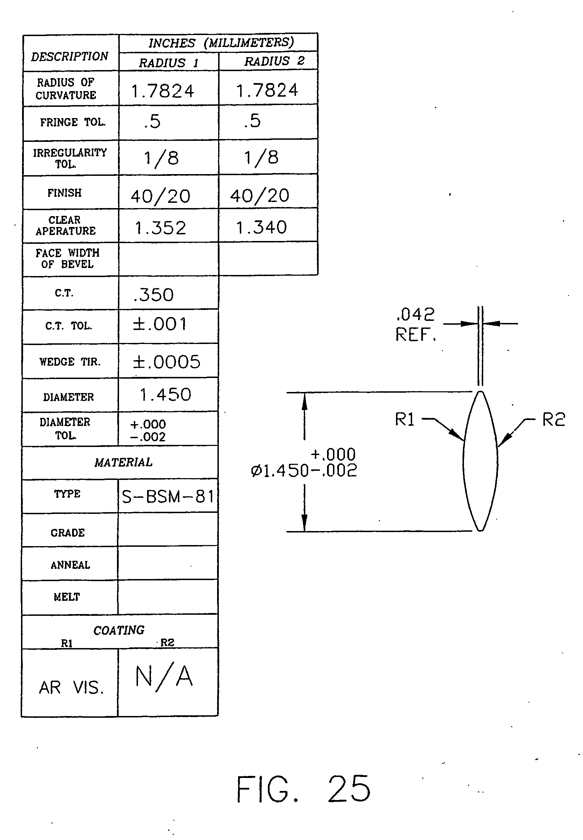

Fig. 25 shows a detailed side plan half cross sectional view with dimensional attributes of the preferred embodiment of element 1 of the lens shown in Fig. 23.

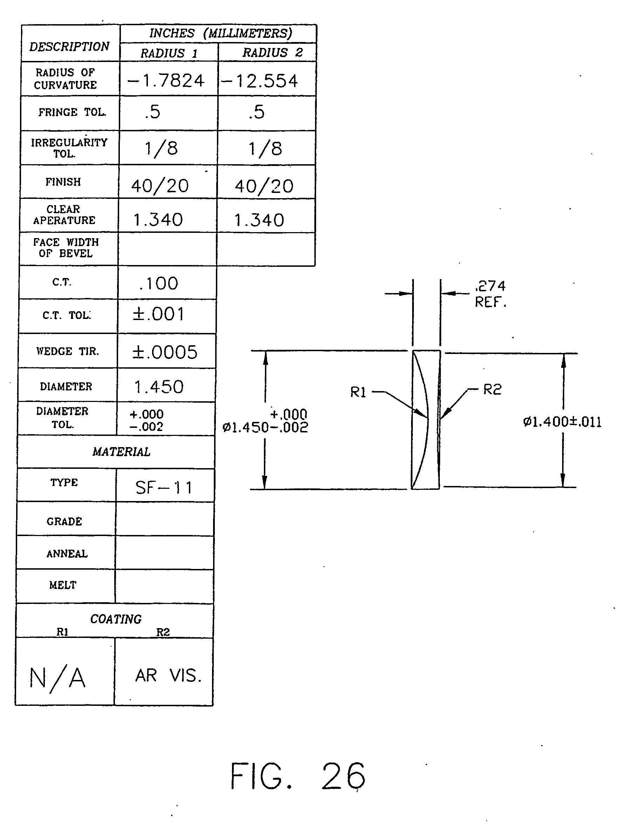

Fig. 26 shows a detailed side plan half cross sectional view with dimensional attributes of the preferred embodiment of element 2 of the lens shown in Fig. 23.

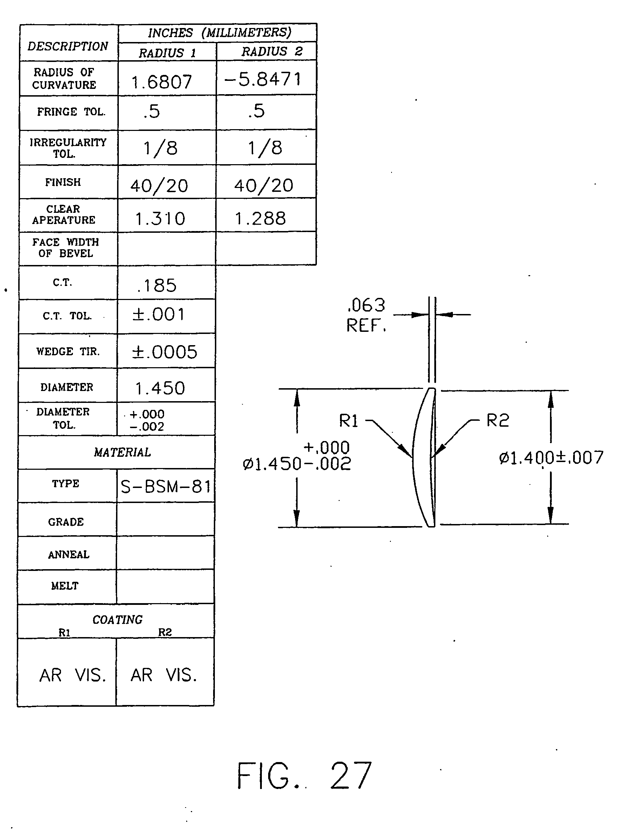

Fig. 27 shows a detailed side plan half cross sectional view with dimensional attributes of the preferred embodiment of element 3 of the lens shown in Fig. 23.

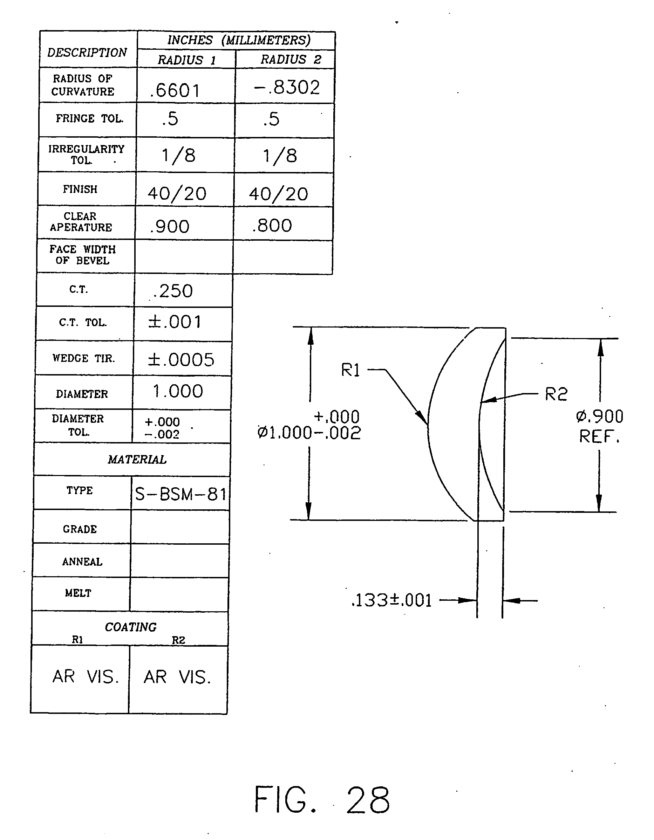

Fig. 28 shows a detailed side plan half cross sectional view with dimensional attributes of the preferred embodiment of element 4 of the lens shown in Fig. 23.

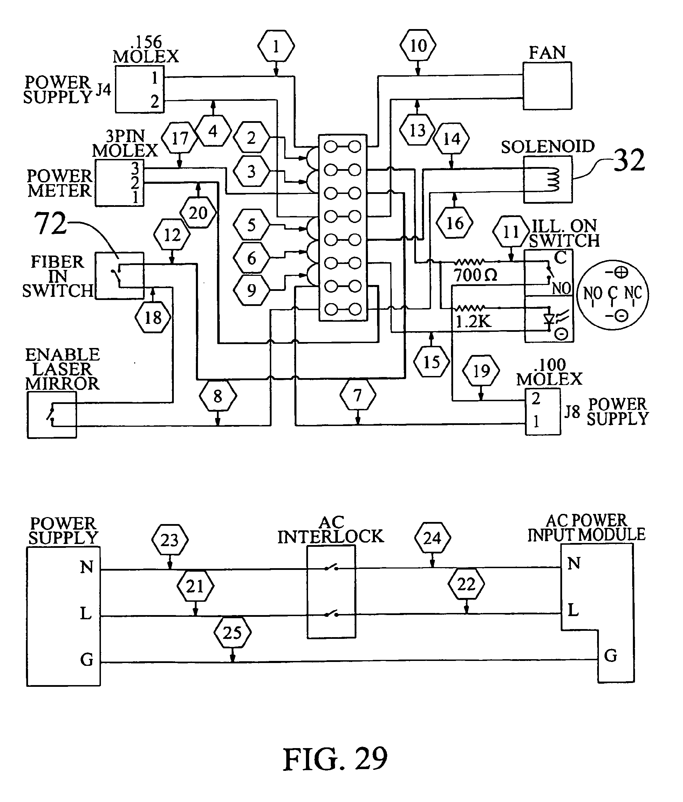

Fig. 29 shows an electrical schematic diagram of the coaxial illuminated laser endoscopic probe and active numerical aperture control apparatus.

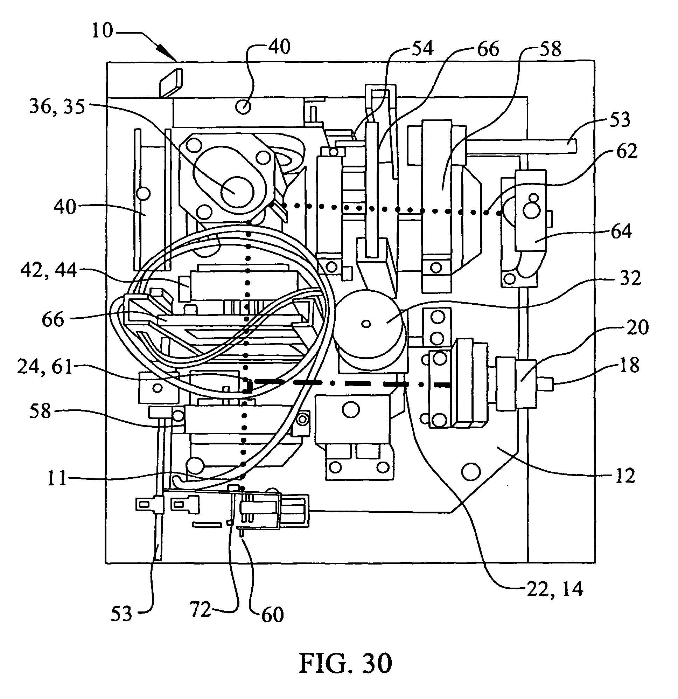

Fig. 30 shows a top perspective view in black and white photographic form of a preferred embodiment of the coaxial illuminated laser endoscopic probe and active numerical aperture control apparatus showing illumination and laser light paths without the phototoxicity card, power meter, and ferrule connectors.

### DETAILED DESCRIPTION

Referring now to the drawings, there is shown in the Figures both preferred and alternate embodiments of the coaxial illuminated laser endoscopic probe and active numerical aperture control apparatus **10** also herein described as an illumination and laser source **10**. There is provided a device **10** for providing non-coherent illumination light **11, 62** and coherent laser treatment light **14** through a single optical fiber **60** of the size typically used for laser treatment only in a safe, effective, and user friendly manner. The apparatus is especially suited for use during ophthalmic surgery.

The present art, in a preferred embodiment, utilizes a 75 watt xenon arc lamp **36** for its high luminance illumination (light density), greater than 6000 °K color temperature, and greater than 95 color rendering index. A unique and useful feature is the very high luminance and small size plasma ball formed on the end of the lamp **36** cathode. If imaged correctly the plasma ball is bright enough to provide the required illumination input to a small fiber such as that used for laser treatment. The xenon arc lamp **36** further provides an extremely small point light source which allows for a smaller output illumination beam **37** diameter. Unique to the present lamp source is a mount **38** which allows for replacement of the lamp **36** and yet retains the location of the plasma ball of said source **36** precisely at a predetermined location within the optical center **35** of the apparatus.

A classic spherical reflector **40** and two lens **42, 58** light collection layout is utilized rather than other lower part count layouts, such as using an elliptical reflector or a combination of a parabolic reflector and lens. This technique allows maximum collection efficiency with a minimum of geometric aberration. The lamp **36** is located at the geometrical center **35** of the reflector **40** and at the focus (focal point) of the first lens **42**. Light that is incident on the reflector **40** is reflected back to the lamp **36**. This forms an upside down or inverted image of the source **36** coincidental to the source **36**. The first lens **42** collimates light from the source **36** and the upside down or inverted image. The second lens **58** is located coaxial to the first lens **42** and focuses the light at its focal point. The output optical fiber **60** is located at the focal point of the second lens **58**. The aforesaid reflectors **40** are preferably spherical rather than parabolic in order to reflect illumination light in the same form as sourced from the arc lamp **36**.

Best form lenses **42**, **58** (plano convex aspheric, facing each other) are used in the present art. It was discovered that chromatic aberrations, caused by the lenses, gave the output of the optical fiber **39**, **60** either a yellow or blue cast. This is not a problem with other ophthalmic sources because the source is many times larger than the output optical fiber. A color corrected "f l" or possibly .5 numerical aperture lens set **42**, **58** consisting of four elements was designed to be utilized for each lens. Each of the elements is coated with a MgF (magnesium fluoride) anti-reflective coating to minimize light losses, with other anti-reflective coatings or layers also utilizable. Use of the achromatic lens sets allows a high fidelity image of the illumination source **36** to be focused onto the end of the optical fiber **60**, **64**. That is, the multi-element lenses allow for a minimum of chromatic aberration. The aforesaid four element lens set is shown and specifically described in the Figures.

An additional separate illumination path **62** is possible with the present art. A 0.5 system numerical aperture or "f 1" lens is the greatest practical because of limitations to the numerical aperture of available optical fibers. This equates to 60 degrees full angle. When the spherical reflector **40** is considered, an additional 60 degrees is provided from the total of 360 degrees available. Consideration of the vertical rotation around the source **36** is impractical because of shadows caused by the lamp **36** electrodes. A total of 240 degrees of horizontal rotation around the lamp 36 are left unaccounted for. Allowing for optics mounts **44** does account for some additional amount. However, at least half of the illumination output is available. This leaves room for a second light path **62** located orthogonal to the first path **11** along with the second fiber output **64**. No other conventional illumination light source incorporates multiple light paths from a single lamp, that is two independent collection systems for illumination light. The independent nature of the two paths **11, 62** allow different filtering and intensity control settings to the two outputs **39, 41**.

Output dimming of the present art illumination system is accomplished by steering the first (collimating) or penultimate lens **42** in a fashion that does not change the lens **42** numerical aperture or introduce shadow artifacts into the beam **37**. The lens set mount **44** has two halves **46** and a flat spring **52**. The first part **48** is attached to the optics bench **12**, the second part **50** holds the lens set **42**, and the spring **52** connects the two **48, 50** together on one side. Pressure on the lens mount second part **50** causes the spring **52** to deflect and the lens **42** to move in a direction generally perpendicular to the optical axis. This results in motion or movement of the image across the face of the optical fiber **60, 64** whereby the peak illumination of the beam **37** is not centered on the optical fiber **60, 64** face during dimming. Due to the aforesaid, the reduction of the output light from the fiber **60, 64** without affecting the color (i.e. color temperature) or aperture of the output is achieved. In a preferred embodiment a shaft mounted cam **54** applies the pressure to the lens mount second part **50** and spring **52**. A control knob **56** is attached to the other end of the shaft **53** and allows the user to select the desired illumination level by rotating the knob **56**. This method is capable of providing at least 95% reduction in output illumination intensity. In a preferred embodiment, a shutter **57** is mounted upon the shaft **53** and is rotated across the illumination beam **37** in order to fully attenuate the output illumination intensity upon full rotation of said knob **56**. Alternative embodiments may utilize other methods, including but not limited to electric or electronic drives, to rotate said shaft **53** instead of said knob **56**.

A dichroic "hot" mirror filter **66** is placed in the collimated space **61** between the illumination lenses **42, 58**. This provides both UV and IR filtering of the light. Brackets are attached to the hot mirror **66** mount to provide a means for additional user selectable filters. Positioning of the filters is critical because this is the only area where the light 11 is generally normal to the filter surface. Location of the filter **66** on the other sides of the lenses would cause the light to have many undesirable incidence angles (between 0 and 30 degrees). Variation in the incidence angle causes dichroic reflectors or filters to have a shift in their affect. If absorption filters are used, placement outside the collimated space **61** will cause an increase in reflective losses and heating problems.

The output optical fiber connector **98** is uniquely configured to provide the precise positioning required while reducing cost. A precise connector or mating end **116** is combined with an integral retention thread **130** to reduce parts cost and assembly time. An optional groove or recess **148** is placed on a second version of the connector to provide for sensing the difference between illumination only and laser compatible output fibers. Placement of a smooth diameter connector **74** into the output activates a switch **72** which will allow the laser power to be mixed. Either the lack of a connector **98** or the groove or recess **148** under the switch **72** will cause the switch **72** to not activate and the laser power will not be mixed in.

Regarding mixing of laser treatment energy or light **14**, laser light **14** is delivered to the system via a preferably 50 micron optical fiber **16** or equivalent. The connector **18** on the laser end is configured to be compatible with the laser and to provide the necessary interface to signal to the laser that a fiber is connected. The laser and light source **10** end preferably uses an SMA 905 connector or equivalent to allow repeatable connections of the laser delivery fiber **16**. Laser light **14** exiting the delivery fiber is preferably collimated using a 16 mm focal length achromatic lens or equivalent **20**, i.e. laser collimating lens, which can also be utilized to focus the collimated laser beam **22**. The position of the fiber **16** is adjusted to be at the focal point of the lens **20**. The input laser connector **18** and collimating lens **20** are located so that the collimated beam **22** is orthogonal to and intersects the center of the illumination axis **11** between the illumination lens sets **42, 58** (the collimated area for illumination light). If all safety requirements are met (i.e. laser output compatible fiber inserted and selection switch for laser output activated) a steering mirror **24** reflects the collimated laser light **22** into the center of the illumination axis **11**. The steering mirror **24** is a first surface plano that is positioned at **45** degrees to the laser light **14** and is located in the center of the illumination axis **11** (when laser mode is active). A unique aspect of the present invention is that the thickness of the mirror **24** is shaped to appear as a circle when viewed along the illumination axis. Due to the **45** degree surface orientation, the shaping causes the mirror **24** surface to appear elliptical when viewed from a normal angle. The size of the mirror **24** is chosen to be minimally larger that the collimated laser beam **22**. Placement of the steering mirror **24** in the center of the illumination axis **11** causes the light rays that would normally be there to be blocked and a shadow to appear in the center of the output light cone. The second illumination lens **58** focuses the laser light **14** reflected by the steering mirror **24** onto the end of the output fiber **60, 64**. Because the length of the output optical fiber strand is relatively short, the incidence angle of light entering the input end is very nearly the same angle on the output end. This results in the output of the fiber strand having a cone of white light with a shadow in the center nearly filled with the laser aiming beam (treatment beam during treatment). That is, the laser provides an aiming beam, typically red, when not fully activated for treatment and a treatment beam, typically green, when fully activated. Without the shadow caused by the steering mirror **24** the aiming beam would be entirely washed out or imperceptible except at very low illumination levels.

Alternate embodiments may utilize more than one steering mirror **24** or place the steering mirror **24** outside of the illumination axis or illumination light path **11** and direct the laser light **14** through an aperture **158** in said spherical reflector **40** and thereafter through the arc lamp **36** plasma ball or through a dichroic reflector **160** or a reflector having an aperture **162**. All of the aforesaid alternate embodiments place the laser light **14** within the collimated space **61** and utilize the second lens **58** for focus upon the output optical fiber **60**. Moreover, all of the aforesaid alternate embodiments provide for a second light path **62** output as seen in the Figures.

The laser steering mirror **24** is mechanically mounted on a thin post **28** that holds it in place while minimizing the loss of illumination light **11**. The post **28** is mechanically connected to a bracket **30** which is connected to a solenoid **32**. The solenoid **32** causes the bracket **30** and also the steering mirror **24** to move into one of two positions. Position one is outside the collimated illumination and laser light. This position is used for no laser delivery and allows the illumination path to operate unaffected. Position two is with the steering mirror **24** located to reflect the laser light into the illumination path **11**. Motion of the solenoid **32** and bracket **30** are controlled by a precision ball slide **34**. Use of the slide **34** insures repeatable positioning of the mirror **24**.

As described, unique to the present art is a coaxial laser and illumination path apparatus **10** which heretofore has not be available or utilized. Also unique to the present art is a highly efficient illumination system which utilizes spherical reflectors **40** and associated lenses **42, 58** to capture a maximum light output and also provide a twin path illumination light output from a single lamp source in order to feed fibers of diameter less than 500 microns. Further unique to the present art is a laser or steering mirror **24** having a solenoid **32** selectability which provides an aiming hole within the illumination path **11** for laser placement. Still further unique to the present art is an illumination arc lamp **36** system having an extremely small point light source **36** which allows for an extremely small illumination focus size or numerical aperture output. Also unique to the present art is an arc lamp **36** mount **38** which precisely and interchangeably places the plasma ball of the arc lamp **36** at the focus or optical center **35** of the optics system. Also unique to the present art is a unique dimming mechanism which moves the focal point of an output dimming or first lens **42** in order to provide dimming without introducing artifacts, chromatic aberrations, or change of color temperature. Also unique to the present art is a capability of connection with existing conventional laser light sources whereby laser treatment and illumination are both provided at an output of the present art apparatus **10**. The optical system of the present apparatus **10** is uniquely capable of accepting the input cone angles of the illumination **33** and laser light **15** placed at the output optical fiber **60** and substantially reproducing said cone angles at the output of the optical fiber, typically where the endoscopic probe is located, with any aberrations caused by the optical fiber itself.

Further alternate embodiments of the present art apparatus **10** may utilize parabolic reflectors instead of spherical reflectors in order to collimate the illumination source **36**. This technique would eliminate the need for the first collimating lens **42** and allow transmission of the laser beam **22** through an aperture within the parabolic reflector or via a steering mirror **24** within the collimated space **61**. Still further alternate embodiments may utilize an elliptical reflector having two focal points whereby the illumination source **36** is placed at the first focal point and the output fiber **60** is placed at the second focal point with the laser beam **22** introduced through an aperture within the elliptical reflector or via a steering mirror **24** between the illumination source **36** and the output fiber **60**. This latter alternate embodiment requires focusing the laser beam **22** onto the output fiber **60** via a lens placed within the laser beam path **22** prior to the output fiber **60** yet allows elimination of both the first collimating lens **42** and the second focusing lens **58**.

Some of the variables which determine the phototoxicity risk level during vitreoretinal surgery include the spectral and power characteristics of the light source used, the type and size of the endoilluminator probe, the length or duration of the surgical procedure, and the area (size) of the illuminated tissues. In each case the surgeon must make a risk-benefit judgement about the intensity of light to be used. Use of insufficient intensity may result in inadequate visualization and adverse effects more serious than a retinal photic injury. Currently, the calculation of the exposure time required to reach a point of injury is a tedious chore involving the numerical integration of the spectral power density function of the light source **36** with a hazard function (see ISO 15752), and specific knowledge of the surgical illumination area and endoilluminator characteristics.

The present art further represents a novel apparatus and method for providing the ophthalmic surgeon with graphical photoxicity risk information in a clear and easy to understand manner. In a preferred embodiment, an inexpensive photoxicity risk card **76** is removably attached to the control panel of the surgical illumination and laser light source **10**. Preferably, the present art card **76** is attached in close proximity to the light intensity control knob **56** in order to show the relationship between the output intensity of the light source and the likelihood of photic injury. The card **76** is preferably included with each endoilluminator instrument, i.e. optical fiber, that is calibrated to represent the phototoxic performance of that instrument type when used with a particular type of light source. The graphical representation **78** on the card **76** acts as a guide for adjustment of the output intensity of the source **10** in relationship to an accepted standard, that is such as the "Millennium" from Bausch and Lomb®. In this way the spectral and power characteristics of the various elements involved in delivering light to the eye are integrated into a single and easily manageable variable. This greatly reduces the complexity of judging the best intensity to use in a given situation. Alternative embodiment graphical representations **78** could present other information regarding the light output such as lumen output (a unit that is weighted by the photopic response of the eye). Other representations could present threshold information when used with special dyes or colored light filters.

An embodiment comprises a card **76** that is die-cut from white chipboard stock that is approximately the weight of a business card. The shape of the card **76** is generally square with a slot **90** removed from one side to enable the card **76** to be placed behind the intensity control knob **56** of the illumination and laser source **10** while providing clearance for the control shaft **53** which is turned by said knob **56**. In a preferred embodiment, four location pins **92** are attached to the front panel of the illumination and laser source **10** enclosure. The pins **92** provide boundaries for card 76 location and tend to inhibit rotation of the card **76** with the control knob.

In a preferred embodiment, onto the face of the card is printed a circular shaped scale **84** that has different color bands **86** representing the phototoxicity risk at a given intensity level, for example green, yellow, and red. The control knob **56** has an indication line that points to the current output intensity level and concurrent phototoxicity risk associated with the probe being used. Unique to the present art is the ability of the card **76** to indicate output intensity at the optical fiber output. The card **76** is meant to be disposed of after a single use and replaced with a new one provided with each optical fiber instrument. In this manner the output of the light source **10** is recalibrated each time it is used. The calibrated unit type may vary with different instrument styles to provide the surgeon with the most pertinent information possible.

As aforesaid the card **76** provides a known point of reference relative to the prior art illumination devices. For example, if the surgeon maintains the knob **56** indicator line within the green color band, he or she will understand that the light intensity output is within the safe intensity of the prior art illuminators such as the "Millennium" from Bausch and Lomb®. This control phenomena is especially useful when utilizing more powerful illumination sources **10** such as described herein. That is, the surgeon must have a prior art point of reference when utilizing more powerful and modem illumination systems such as the present art. The art of the present invention may further provide several bands which do not provide a reference to the prior art but instead indicate phototoxicity levels or light intensity levels directly to the surgeon.

Unique to the present art is the ability of the manufacturer of the optical fiber to provide a phototoxicity risk card **76** which accounts for attenuation and spectral absorption within the optical fiber provided with said card **76**. Thus for example, if an optical fiber is highly attenuating, the card may indicate that the surgeon must turn the intensity control knob **56** to a higher level in order to obtain an equivalency to one or more of the aforesaid prior art illuminators or to achieve a desired photo-illumination output.

The art of the present device also comprises a ferrule or connector **98** having an internal bore **102**, preferably stepped **104**, which is substantially parallel with the lengthwise axis **100** of the ferrule body **98**. The aforesaid bore **102** allows for placement and bonding or potting of an optical fiber within and through said ferrule body **98**. Externally, said ferrule body **98** is also stepped **112, 148** in a unique form in order to optimally function as described herein.

In a preferred embodiment, the ferrule body **98** has an external end **114** and a mating end **116** and externally comprises a substantially cylindrical head **118** of a first diameter **120** having a first end **122** and a second end **123**, said first end **122** co-located with said external end **114**. Said ferrule body **98** further externally comprises a lip **124** of greater diameter than said head **118** and having a first side **126** and a second side **128** with said first side **126** mounted with said second end **123** of said head **118**. A threaded portion **130** of preferably smaller diameter than said head **118** is attached with and extends from said lip **124** second side **128**. In a preferred embodiment, said threaded portion **130** first comprises an 8-32 UNC thread with a first end **132** and second end **134**, said first end **132** connected with said second side **128** of said lip **124**. Also in a preferred embodiment, said threaded portion **130** has a groove **136** of approximately .030 inch at said first end **132** with approximately .090 inch of said thread **130** thereafter following and another approximately .030 inch groove **136** following said thread **130** at said second end **134**. Externally the ferrule body **98** also has an alignment barrel **138** having a first **140** and second **142** end following said threaded portion **130**, said first end **140** attached with said threaded portion **130**. The second end **142** of said alignment barrel **138** is co-located with said mating end **116** of said ferrule body **98**. Also, said second end **142** of said alignment barrel **138** contains an orifice **144** of substantially equivalent or slightly greater diameter as the optical fiber mounted within said stepped bore **102**. Said orifice **144** is interconnected with said internal stepped bore **102**. In an embodiment of the present art, said orifice is approximately .011 inch in diameter and .025 inch in length. Also in a preferred embodiment, said alignment barrel **138** has a chamfer **146** at the circumference of said second end **142**. Preferably said chamfer **146** is of approximately 45 degree angle and .015 inch in length. Alternative embodiments may utilize chamfers of different angles or shapes or forego use of a chamfer altogether.

The alignment barrel **138** of the present art is uniquely shaped within the embodiments to indicate whether laser light or illumination light should be applied to the optical fiber. In a preferred embodiment of the laser ferrule, the alignment barrel is of uniform diameter, approximately .118 diameter, which indicates to the source **36** that laser light or energy is desired. In a first alternative embodiment or illumination ferrule, the alignment barrel contains a recess **148** located approximately .075 inch from said barrel **138** second end **142** and extending approximately .268 inch from said second end **142**. When utilized, the illumination and laser source **10** detects this recess and determines that illumination light and not laser light is desired. Further alternative embodiments may utilize the aforesaid recess **148** embodiment for laser light and the uniform barrel diameter for illumination light.

Internally said stepped bore **102** first comprises a first larger bore substantially within said head portion which is of approximately .098 inch diameter and extends substantially the length of said head. A second intermediate bore of approximately .063 inch diameter extends from said first larger bore to said orifice **144** within said threaded portion **130** and said alignment barrel **138**. Also in a preferred embodiment, the orifice **144** length is approximately .025 inch. Alternative embodiments may utilize first and second bores and orifices having a plurality of diameter and length sizes provided that the diameter portions are smaller than the ferrule external portions within which each is located.

When assembled with an optical fiber, the optical fiber extends through said bore **102** and orifice **144** and terminates substantially flush with said ferrule body **98** mating end **116** or second end **142** of said alignment barrel **138**. Preferably said optical fiber is held within said bore **102** via potting or adhesive compounds surrounding said fiber and attaching with said bore **102** of the ferrule **98**.

In a preferred embodiment, the external head **118** diameter is approximately.234 inch with a length of approximately .375 inch. The lip **124** external diameter is approximately 312 inch with a thickness of approximately .025 inch. Also, said alignment barrel **138** is approximately 118 inch in diameter and .380 inch in length.

Where provided, dimensions, geometrical attributes, and thread sizes are for preferred embodiment informational and enablement purposes. Alternative embodiments may utilize a plurality of variations of the aforesaid without departing from the scope and spirit of the present invention. This is especially true as relating to said head **118**, lip **124**, and threaded portion **130**. Said lip **124** may be integrated as part of the head **118** or removed completely. Also, the position, location, and type of threaded portion **130** may vary. Said threaded portion **130** may not utilize said grooves **136**, utilize grooves of a shorter or longer length, or have said head **118** and lip **124** diameters sized substantially the same as or smaller than the outside diameter of said threads **130**. The art of the present device may be manufactured from a plurality of materials, including but not limited to metals, plastics, ceramics, or composites.

Unique to the present device is the integral inclusion of a laser power meter **150** having a sensor **152**, a power display **154**, and associated control circuitry **156**. The power meter **150** allows a surgeon to place the endoscopic fiber optic probe onto said sensor **152**, energize the laser through the illumination and laser source **10** and measure the laser power output as seen on said display **154**. Inclusion of the aforesaid is especially useful due to variations in optical fibers or to account for attenuation through the illumination and laser source **10**. By utilizing the power meter **150**, the surgeon has complete knowledge of the laser power transmitted to the surgical site. Alternative embodiments may utilize said power meter **150** for measurement of the output illumination **37** intensity as well as the laser light **14** power.

In operation, the surgeon connects a laser light source via optical fiber to the input laser connector **18** on the apparatus **10**. The surgeon thereafter connects a ferrule connector **98** with an integral optical fiber connected with an endoscopic probe at the first output **39** or for illumination only at said second output **64**. If said ferrule connector **98** at said first output **39** does not have the aforedescribed recess **148**, the apparatus **10** will allow the steering mirror **24** to position within the illumination light path **11** and further allow transmission of laser light. If the surgeon desires to measure laser power output, he or she places the output end of the endoscopic probe onto said sensor **152** and upon full laser activation, reads the laser power output on the display **154**. If the apparatus **10** is powered, the surgeon proceeds to illuminate the tissues of concern with a cone of white illumination light having a shadow where the laser beam will be placed and a typically red laser aiming beam within said shadow. Upon full activation of laser power, a typically green treatment laser beam replaces said typically red aiming beam to treat the tissues of concern. All of the aforesaid illumination and treatment may be achieved with a single incision and through a single optical fiber of smaller diameter than prior art sources.

Those skilled in the art will appreciate that a coaxial illuminated laser endoscopic probe and active numerical aperture control apparatus **10** (illumination and laser source) and method of use such has been shown and described. The apparatus and method of use allows for simultaneous transmission of illumination and laser treatment light through a single optical fiber of a size which is typically utilized for laser treatment light only. The apparatus and method further provides control of the angular light output from the endoscopic probe attached with said optical fiber. The apparatus also provides for distinct and separate illumination without utilization of the treatment laser while providing complete intensity control of said illumination. Those skilled in the art will appreciate that a medical light intensity phototoxicity control or risk card **76** has also been shown and described for use with the present art. Said phototoxicity risk card **76** is especially useful for quick and easy determination of illumination intensity output from a specific type of optical fiber or higher power source such as the present art. Those skilled in the art will appreciate that a photon illumination and laser ferrule connector **98** has also been shown and described. Said ferrule **98** is especially useful for quick and positive connection of an optical fiber to a laser or illumination source **10** as herein described and further allows said source **10** to distinguish the optical fiber type or use, that is for illumination or medical laser application. The present art device is useful during surgery and especially ophthalmic surgery. Also, those skilled in the art will appreciate the integral inclusion of a laser optical fiber output power meter.

Having described the invention in detail, those skilled in the art will appreciate that modifications may be made of the invention. Therefore, it is not intended that the scope of the invention be limited to the specific embodiments illustrated and described. Rather it is intended that the scope of this invention be determined by the appended claims and their equivalents.

## Claims

1. An illumination source comprising:
a single illumination source having an output of illumination light (11) transmitted through one or more lenses (42) and focused upon an optical fiber (60); and
a dimming mechanism comprising a mount (44) on one or more of said lenses (42) which is capable of steering one or more of said lenses (42) whereby a peak illumination of said illumination light (11) is not centered on said optical fiber (60) during dimming.

## Patentansprüche

1. Beleuchtungsquelle mit
einer einzigen Beleuchtungsquelle, die einen Ausgang von Beleuchtungslicht (11) hat, das durch eine oder mehrere Linsen (42) übertragen und auf einer optischen Faser (60) fokussiert wird, und
einem Dimmmechanismus mit einer Halterung (44) an einer oder mehreren der Linsen (42), die in der Lage ist, eine oder mehrere der Linsen (42) zu steuern, wobei eine Spitzenbeleuchtung des Beleuchtungslichts (11) während des Dimmens nicht auf der optischen Faser (60) zentriert ist.

## Revendications

1. Source d'éclairage comprenant:
une source d'éclairage unique ayant une sortie de lumière d'éclairage (11) transmise par l'intermédiaire d'une ou plusieurs lentilles (42) et focalisée sur une fibre optique (60), et
un mécanisme d'atténuation ayant une monture (44) sur une ou plusieurs desdites lentilles (42), qui est apte à diriger une ou plusieurs desdites lentilles (42), dans lequel une illumination de pointe de la lumière d'éclairage (11) n'est pas centrée sur la fibre optique (60) pendant l'atténuation.
